# EUROPÄISCHE PATENTSCHRIFT

(11) **EP 3 893 820 B1**
(45) Veröffentlichungstag und Bekanntmachung des Hinweises auf die Patenterteilung: **30.09.2026**
(21) Anmeldenummer: 19824259.6
(22) Anmeldetag: 11.12.2019
(51) Int. Cl.: A61F 5/01

(54) **GELENK FÜR EINE KNÖCHELORTHESE**
JOINT FOR AN ANKLE ORTHESIS
ARTICULATION D'ORTHÈSE DE CHEVILLE

(30) Priorität: 12.12.2018 DE 102018131929
(43) Veröffentlichungstag der Anmeldung: 20.10.2021
(73) Patentinhaber: Ottobock SE & Co. KGaA, 37115 Duderstadt (DE)
(72) Erfinder: LÜRSSEN, Marcus, 37073 Göttingen (DE); MÜLLER, André, 37115 Duderstadt (DE); SCHIMEK, Norbert, 37339 Kirchworbis (DE); JUNG, Marcel, 37115 Duderstadt (DE); KROLL-ORYWAHL, Olaf, 37154 Northeim (DE)
(74) Vertreter: Gramm, Lins & Partner Patent- und Rechtsanwälte PartGmbB
(86) Internationale Anmeldenummer: PCT/EP2019/084615
(87) Internationale Veröffentlichungsnummer: WO 2020/120559

(56) Entgegenhaltungen:
- WO-A1-2016/201318
- DE-U1- 29 902 609
- US-A1- 2006 173 392
- US-A1- 2008 082 031
- US-A1- 2018 289 523

## Beschreibung

Die Erfindung betrifft ein Gelenk für eine orthopädietechnische Einrichtung, wobei das Gelenk ein erstes Element mit wenigstens einem Anschlagselement und zweites Element aufweist, das an dem ersten Element verschwenkbar gelagert ist und wenigstens eine Kontaktfläche aufweist, die mit dem wenigstens einen Anschlagselement in Kontakt bringbar ist, indem das zweite Element relativ zu dem ersten Element verschwenkt wird, wobei das wenigstens eine Anschlagselement dann einem weiteren Verschwenken des zweiten Elementes relativ zum ersten Element eine Kraft entgegensetzt, wobei die orthopädietechnische Einrichtung eine Knöchelorthese ist, wobei das erste Element ein Unterschenkelelement und das zweite Element ein Fußbügel ist, wobei das Anschlagselement wenigstens ein Federelement und/oder wenigstens ein Dämpferelement aufweist und die Kraft überwindbar ausgestaltet ist, so dass ein weiteres Verschwenken möglich ist.

Ein derartiges Gelenk für eine Knöchelorthese ist beispielsweise aus der DE 10 2010 014 334 A1 bekannt. Andere Gelenke für Orthesen sind aus der DE 299 02 609 U1 oder der US 2008/0082031 A1 bekannt. Bei dem Gelenk, das in der US 2018/0289523 A1 beschrieben wird, werden Anschlagsstifte in das Gelenk zwischen die beiden Gelenkteile geschoben, die als Anschlag dienen. Andere Ausführungen von Gelenken sind der US 2006/0173392 A1 und der WO 2016/201318 A1 zu entnehmen.

Unter einer Knöchelorthese werden vorliegend alle Orthesen verstanden, die den Knöchel einer unteren Extremität überbrücken. Unter einer Knöchelorthese werden vorliegend auch Bein- und Unterschenkelorthesen verstanden, sofern sie auch einen Fußteil haben und somit den menschlichen Knöchel überbrücken.

Bei derartigen Knöchelorthesen ist das erste Element normalerweise ein Unterschenkelelement und das zweite Element ein Fußbügel. Selbstverständlich ist auch die umgekehrte Ausgestaltung möglich. Das Unterschenkelelement ist vorzugsweise am Unterschenkel des Trägers der Knöchelorthese festzulegen. Dafür kann es beispielsweise einen Gurt aufweisen, der um den Unterschenkel herum gelegt wird. Das Unterschenkelelement des Gelenkes kann jedoch auch lediglich ein Befestigungselement sein, an dem ein weiteres Unterschenkelelement, beispielsweise eine Unterschenkelschiene, angeordnet werden kann. Der Fußbügel kann einstückig mit einem Fußteil, das beispielsweise unter der Sohle des Fußes des Trägers der Knöchelorthese angeordnet wird, ausgebildet sein oder lediglich ein Befestigungselement sein, an dem ein entsprechendes Fußteil angeordnet werden kann.

Aus therapeutischer Sicht kann es sinnvoll sein, die Länge der Schwenkbewegung, also den maximal möglichen Schwenkwinkel zwischen dem Unterschenkelelement und dem Fußbügel zu begrenzen und beispielsweise in einer oder beiden Richtungen einen Anschlag vorzusehen. Um ein zu hartes Anschlagen dieser Anschläge zu vermeiden, sind die Anschläge oftmals federbelastet und damit gedämpft ausgeführt. Insbesondere in diesen Ausgestaltungen, jedoch auch ohne mit Anschlägen ausgestattet zu sein, sind Knöchelorthesen und insbesondere deren Gelenk im Gebrauch beim Gehen großen mechanischen Belastungen ausgesetzt. Dadurch kann es zu Verschleiß, Beschädigungen und Fehlfunktionen kommen.

Im angelegten Zustand der orthopädietechnischen Einrichtung wird das zweite Element relativ zu dem ersten Element verschwenkt, wenn beispielsweise der Träger der orthopädietechnischen Einrichtung eine bestimmte Bewegung macht, beispielsweise ein Knie beugt oder einen Schritt macht. Dabei kommt bei einer bestimmten Position des ersten Elementes relativ zu dem zweiten Element die Kontaktfläche des zweiten Elementes mit dem Anschlagselement in Kontakt. Ein weiteres Verschwenken in die gleiche Richtung hat eine dem Verschwenken entgegengesetzte Kraft zur Folge. Diese Kraft kann bei einem federbelasteten oder gedämpften Anschlagselement überwindbar ausgestaltet sein, so dass ein weiteres Verschwenken möglich ist. Bei einem festen oder statischen Anschlag ist die Kraft unüberwindbar, ohne das Gelenk zu beschädigen. Diese Kraft wird von dem Anschlagselement aufgebracht.

Der Erfindung liegt die Aufgabe zugrunde, diese Nachteile zu beheben oder zumindest abzumildern.

Die Erfindung löst die gestellte Aufgabe durch ein Gelenk für eine orthopädietechnische Einrichtung gemäß dem Oberbegriff des Anspruchs 1, das sich dadurch auszeichnet, dass an dem zweiten Element wenigstens ein Kontaktelement lösbar befestigt ist, das die wenigstens eine Kontaktfläche aufweist.

Die Stelle, an der die von dem wenigstens einen Anschlagselement aufgebrachte Kraft, in das zweite Element eingebracht wird, wird durch die erfindungsgemäße Ausgestaltung durch die Kontaktfläche des wenigstens einen Kontaktelementes ausgebildet. An dieser Stelle treten gegebenenfalls die aus dem Stand der Technik bekannten Abnutzungserscheinungen, Verschleiß und Beschädigungen auf. Da das wenigstens eine Kontaktelement lösbar am zweiten Element angeordnet ist, ist es leicht auszutauschen, sodass das Gelenk besonders einfach wieder instand gesetzt werden kann. Dazu ist es gegebenenfalls ausreichend, das Gelenk einmal zu demontieren, das wenigstens eine Kontaktelement vom zweiten Element zu entfernen und ein intaktes Kontaktelement wieder lösbar am zweiten Element anzuordnen. Herkömmlicherweise sind am ersten Element und am zweiten Element des Gelenks für die orthopädietechnische Einrichtung individuell angefertigte Orthesenelemente angeordnet, die auf den Träger der Orthese angepasst sind. Um hier eine einmal gewählte Einstellung, Positionierung und Orientierung der unterschiedlichen Bauteile relativ zueinander beim Tragen der Orthese nicht zu verlieren, werden diese Bauteile in der Regel unlösbar mit dem ersten Element und dem zweiten Element verbunden. Dies geschieht beispielsweise dadurch, dass das erste Element und/oder das zweite Element in das entsprechende Bauteil eingegossen oder an das Bauteil angeklebt werden.

Kommt es aufgrund des Verschleißes zu so großen Deformierungen, Beschädigungen oder Fehlfunktionen, dass ein sicherer Betrieb des Gelenkes für die orthopädietechnische Einrichtung nicht gewährleistet werden kann, muss bei orthopädietechnischen Einrichtungen gemäß dem Stand der Technik das zweite Element und damit auch das mit ihm unlösbar verbundene Orthesenelement, das individuell an den Träger angepasst ist, ausgetauscht werden. Dies hat einen hohen Kosten-, Produktions- und Zeitaufwand zur Folge hat und bedeutet zudem, dass die Orthese für den Träger für eine gewisse Zeit, nämlich die Fertigungsdauer des jeweils auszutauschenden Bauteils, nicht zur Verfügung steht. Dies ist weder aus therapeutischer Sicht noch aus Sicht des Tragekomforts von Vorteil. Dieses Problem wird durch die erfindungsgemäße Ausgestaltung gelöst, da lediglich ein Standardbauteil, nämlich das jeweilige Kontaktelement von dem zweiten Element entfernt und durch ein intaktes Kontaktelement ausgetauscht werden muss.

Erfindungsgemäß ist die orthopädietechnische Einrichtung eine Knöchelorthese. Das erste Element ist ein Unterschenkelelement und das zweite Element ein Fußbügel.

In einer bevorzugten Ausgestaltung weist das wenigstens eine Anschlagselement wenigstens ein Federelement und/oder wenigstens ein Dämpferelement auf.

Vorzugsweise sind an dem zweiten Element wenigstens zwei Kontaktelemente lösbar befestigt und das Gelenk weist wenigstens zwei Anschlagselemente auf, deren aufgebrachte Kraft in jeweils wenigstens eine Kontaktfläche wenigstens eines Kontaktelementes eingeleitet wird. Die Anschlagselemente können identisch oder unterschiedlich ausgebildet sein. Besonders vorteilhaft wirken die wenigstens zwei Anschlagselemente in unterschiedliche Richtungen, sodass von dem ersten Anschlagselement eine der Dorsalflexion entgegen gerichtete Kraft aufgebracht wird und von dem zweiten Anschlagselement eine der Plantarflexion entgegen gerichtete Kraft, wenn die Kontaktfläche mit dem jeweiligen Anschlagselement in Kontakt ist. Dadurch, dass für unterschiedliche Anschlagselemente unterschiedliche Kontaktelemente vorhanden sind, ist der benötigte Materialeinsatz beim Austausch besonders gering. Ist beispielsweise nur eines der Kontaktelemente verschlissen oder durch Benutzung unbrauchbar geworden, muss lediglich dieses eine Kontaktelement ausgetauscht werden, während das andere Kontaktelement, das einem ordnungsgemäßen Gebrauch der Orthese noch nicht entgegensteht, nicht ausgetauscht werden muss.

In einer alternativen Ausgestaltung ist es selbstverständlich auch möglich, nur ein einziges Kontaktelement lösbar an dem zweiten Element anzuordnen, das zwei Kontaktflächen aufweist, in die die Kraft jeweils wenigstens eines Anschlagselementes eingeleitet wird. Beim Austausch des entsprechenden Kontaktelementes muss dann ein größeres Bauteil entfernt und durch ein intaktes Bauteil ersetzt werden.

In einer bevorzugten Ausgestaltung weist jedes Anschlagselement wenigstens ein Kraftübertragungselement mit einem Kontaktbereich auf, der an der Kontaktfläche des jeweiligen Kontaktelementes anliegt, wenn die von dem Anschlagselement aufgebrachte Kraft in die Kontaktfläche des Kontaktelementes eingeleitet wird. Ein derartiges Kraftübertragungselement kann beispielsweise ein Stößel, ein Stift oder ein Dorn sein, der entlang einer Verschieberichtung federbelastet verschiebbar ist, wenn das Anschlagselement federbelastet oder gedämpft ist. An dem dem Anschlagselement abgewandten Ende verfügt ein solches Kraftübertragungselement vorzugsweise über den Kontaktbereich, der an der Kontaktfläche des Kontaktelementes zum Anliegen gebracht wird.

Bevorzugt ist die wenigstens eine Kontaktfläche derart geformt, dass die von dem wenigstens einen Anschlagselement aufgebrachte Kraft unabhängig von einer Position des zweiten Elementes relativ zu dem ersten Element immer senkrecht auf der Kontaktfläche steht. Auf diese Weise wird eine optimale Kraftübertragung gewährleistet und zudem werden Scherkräfte und Querbeschleunigungen vermieden. Dies ist insbesondere aber nicht ausschließlich dann von Vorteil, wenn das verwendete Anschlagselement entlang einer Kompressionsrichtung komprimiert werden soll, um die von ihm aufgebrachte Kraft auszuüben. Handelt es sich bei dem Kraftübertragungselement um den bereits genannten Stift oder Stößel, ist die Kompressionsrichtung vorzugsweise mit der Verschieberichtung des Kraftübertragungselementes identisch. Das Anschlagselement wird beim Verschwenken des zweiten Elementes relativ zum ersten Element durch das zweite Elementkomprimiert, wobei eine Kraft in das Anschlagselement eingeleitet wird. Es hat sich aus vorteilhaft herausgestellt, wenn diese Kraft unabhängig von der Position des zweiten Elementes relativ zum ersten Element und damit auch unabhängig von der Position des zweiten Elementes relativ zum Anschlagselement immer in Kompressionsrichtung des Anschlagselementes wirkt. Dies kann durch geschickte Wahl der geometrischen Form und Kontur der wenigstens einen Kontaktfläche des Kontaktelementes und der Form und Kontur des Kontaktbereiches des Kraftübertragungselementes erreicht werden. Beide Formgebungen werden so aufeinander abgestimmt, dass das gewünschte Ergebnis erreicht ist.

Besonders bevorzugt werden die Form und Kontur der wenigstens einen Kontaktfläche des Kontaktelementes und die Form und Kontur des Kontaktbereiches des Kraftübertragungselementes so aufeinander abgestimmt, dass beide entlang einer Linie, der sogenannten Lastlinie, in Kontakt kommen. Je länger diese Linie ist, desto geringer ist der an jeder einzelnen Stelle wirkende Druck und desto mehr werden Verschleiß und Abnutzung reduziert. Die Lastlinie erstreckt sich vorzugsweise in der gleichen Ebene wie die Schwenkachse des Gelenkes, bei Gelenkes für Knöchelorthesen also vorzugsweise in der Frontalebene.

Vorteilhafterweist ist das wenigstens eine Kontaktelement aus einem härteren Material als das zweite Element, vorzugsweise aus gehärtetem Stahl, hergestellt. Auf diese Weise wird der Verschleiß und die Verformung des Kontaktelementes reduziert, sodass ein Austausch und ein Ersatz eines deformierten und verschlissenen Kontaktelementes nur selten durchzuführen ist.

In einer alternativen Ausgestaltung ist das wenigstens eine Kontaktelement aus einem weicheren Material als das zweite Element, vorzugsweise aus Eladur, hergestellt. In dieser Ausgestaltung ist sichergestellt, dass nicht etwa ein Kraftübertragungselement, durch das die von dem wenigstens einen Federelement aufgebrachte Kraft in die Kontaktfläche des Kontaktelementes eingebracht wird, dem Verschleiß unterworfen ist und sich verformt und unbrauchbar wird. Vielmehr wird durch das weichere Material des Kontaktelementes sichergestellt, dass das Kontaktelement nach dem Verschleiß und von Zeit zu Zeit ausgetauscht werden muss. Auf diese Weise wird ein kompliziertes Austauschen anderer Bauteile, insbesondere eines Kraftübertragungselementes, vermieden.

Bevorzugt enthält das Kontaktelement ein elastisches Material oder ist sogar überwiegend oder vollständig aus einem elastischen Material hergestellt. Dabei kann wenigstens ein Bauteil aus einem härteren Material, hergestellt sein. Dazu kann beispielsweise ein Metallpin in das elastische Material eingesetzt oder eingebracht sein, um eine Verformung zu verhindern.

Bevorzugt ist die Kontaktfläche konkav ausgebildet. Die Kontaktfläche des Kontaktelementes ist dabei bevorzugt in Form einer leichten Rille, insbesondere einer Längsrille ausgebildet. Sie kann dadurch den Kontaktbereich eines Kraftübertragungselementes führen und eine Querbeschleunigung und Querverschiebung des Kraftübertragungselementes verhindern.

Besonders bevorzugt ist der Kontaktbereich konvex ausgebildet und weist vorzugsweise einen Krümmungsradius auf, der einem Krümmungsradius der Kontaktfläche des Kontaktelementes entspricht. Dadurch wird die Führung des Kontaktbereiches des Kraftübertragungselementes an der Kontaktfläche des Kontaktelementes verstärkt. Durch die aufeinander abgestimmten, vorzugsweise identischen Krümmungsradien wird zudem erreicht, dass die Größe der Fläche, an der die beiden Bauteile miteinander in Kontakt kommen, möglichst groß ist, sodass die an einzelnen Punkten wirkenden Kräfte, also insbesondere der Druck an einzelnen Punkten, möglichst gering gehalten wird. Dadurch werden die von dem wenigstens einen Anschlagselement über das Kraftübertragungselement auf das Kontaktelement übertragenen Kräfte auf eine möglichst große Fläche verteilt, sodass der Druck an einzelnen Stellen reduziert wird. Auch dadurch wird einem übermäßigen Verschleiß entgegengewirkt und die Lebensdauer der einzelnen Bauteile, insbesondere des Kontaktelementes, erhöht.

Vorteilhafterweise ist das wenigstens eine Kontaktelement an dem zweiten Element angeschraubt. Dies kann beispielsweise durch eine oder mehrere Madenschrauben geschehen.

In einer bevorzugten Ausgestaltung sind das wenigstens eine Kontaktelement und das zweite Element derart korrespondierend ausgebildet und geformt, dass das Kontaktelement nur in einer einzigen oder in einer begrenzten Anzahl von Orientierungen an dem zweiten Element befestigbar ist. Dies ist insbesondere dann von Vorteil, wenn die Kontaktfläche des Kontaktelementes strukturiert und nicht eben ausgebildet ist. In diesem Fall kann eine fehlerhafte Orientierung eines neu angeordneten Kontaktelementes am zweiten Element zu Fehlfunktionen und ungewünschten Druckspitzen an der Kontaktfläche des Kontaktelementes führen. Zudem wird durch eine beispielsweise asymmetrische Ausführung des Kontaktelementes, um auf diese Weise zu erreichen, dass es nur in einer Orientierung an dem zweiten Element angeordnet wird, der Fertigungs- und Austauschaufwand reduziert.

Vorzugsweise ist das Gelenk derart ausgebildet, dass das Kontaktelement in zumindest einer Orientierung und/oder Position des ersten Elementes relativ zu dem zweiten Element sichtbar ist, ohne das Gelenk zu demontieren. Auf diese Weise kann der Verschleiß einfach überwacht werden, ohne dass dafür das Gelenk demontiert werden muss.

Vorzugsweise verfügt das wenigstens eine Kontaktelement über zwei voneinander beabstandete Schenkel, zwischen denen das zweite Element des Gelenkes angeordnet wird. Die Schenkel verfügen bevorzugt über jeweils eine Bohrung, die mit einer Ausnehmung oder Bohrung des zweiten Elementes in Überdeckung bringbar ist. In diesem Zustand wird ein Stift oder ein Bolzen durch die in Überdeckung befindlichen Bohrungen geschoben oder gesteckt, so dass das wenigstens eine Kontaktelement mit dem zweiten Element verbunden ist. Bevorzugt wird statt eines Bolzens oder eines Stiftes eine Schraube, beispielsweise eine Schaftschraube verwendet. In diesem Fall verfügt zumindest eine der Bohrungen, durch die die Schraube geführt wird, über ein Innengewinde, das mit einem Außengewinde der Schraube korrespondierend ausgebildet ist. Besonders bevorzugt verfügen alle Bohrungen über ein derartiges Innengewinde.

Bevorzugt verfügen die Schenkel des wenigstens einen Kontaktelementes jeweils über mehr als eine Bohrung, die mit einer entsprechenden Anzahl von Bohrungen oder Ausnehmungen im zweiten Element in Überdeckung bringbar sind. Dann können mehr als ein Stift, Bolzen oder mehr als eine Schraube verwendet werden, um das wenigstens eine Kontaktelement mit dem zweiten Element zu verbinden. Natürlich können auch unterschiedliche Elemente, beispielsweise ein Stift und eine Schraube verwendet werden.

Anhand der beiliegenden Zeichnungen wird nachfolgend ein Ausführungsbeispiel der vorliegenden Erfindung näher erläutert. Es zeigen:
- Figur 1 -: die schematische Darstellung eines Fußbügels für ein Gelenk für eine Knöchelorthese gemäß einem ersten Ausführungsbeispiel der vorliegenden Erfindung,
- Figur 2 -: den Fußbügel aus Figur 1 in einer gedrehten Ansicht.,
- Figur 3 -: einen vergrößerten Ausschnitt aus Figur 2,
- Figur 4 -: einen Teil eines Fußbügels in einer dreidimensionalen Ansicht,
- Figur 5 -: die schematische Schnittdarstellung durch einen Teil eines Gelenkes gemäß dem Stand der Technik,
- Figur 6 -: die Darstellung aus Figur 5 für ein Gelenk gemäß einem Ausführungsbeispiel der vorliegenden Erfindung,
- Figur 7 -: die schematische Darstellung unterschiedlicher Ausführungsformen eines entsprechenden Gelenkes,
- Figur 8 -: die schematische Darstellung einer weiteren Ausführungsform in einer Schnittdarstellung und einer Seitenansicht,
- Figur 9 -: die schematische Darstellung eines weiteren Ausführungsbeispiels in zwei verschiedenen Ansichten und
- Figur 10 -: die schematische Darstellung eines Kontaktelementes in verschiedenen Ansichten.

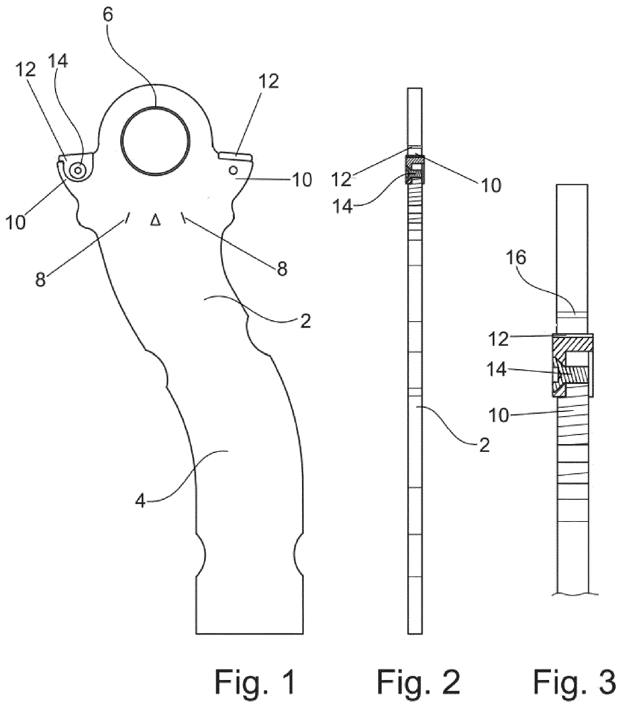

Figur 1 zeigt einen Fußbügel 2 für ein Gelenk für eine orthopädietechnische Einrichtung in Form einer Knöchelorthese gemäß einem Ausführungsbeispiel der vorliegenden Erfindung.

Er verfügt über ein Befestigungselement 4, an dem beispielsweise ein Fußteil einer Knöchelorthese angeordnet werden kann. Dies geschieht vorteilhafterweise unlösbar.

Im oberen Bereich des Fußbügels 2 befindet sich eine Öffnung 6, durch die im montierten Zustand eine Schwenkachse verläuft, um die der gezeigte Fußbügel 2 um ein nicht dargestelltes Unterschenkelelement verschwenkbar ist. Der Fußbügel 2 verfügt über Markierungen 8, die eine optimale Einstellung auf die jeweiligen Bedürfnisse des Trägers der Knöchelorthese erlauben sollen und zudem dafür sorgen, dass einmal eingestellte Einstellungen reproduzierbar sind.

Der Fußbügel 2 verfügt über zwei Schultern 10, an denen jeweils ein Kontaktelement 12 angeordnet ist. In dem gezeigten Ausführungsbeispiel ist das Kontaktelement 12 an der Schulter 10 durch eine Schraube 14 lösbar befestigt.

Figur 2 zeigt den Fußbügel 2 aus Figur 1 in einer Seitenansicht. Man erkennt auf der Schulter 10 das Kontaktelement 12, das mit der Schraube 14 befestigt ist.

Figur 3 zeigt einen vergrößerten Ausschnitt. Deutlich zu erkennen ist die Schraube 14, mit der das Kontaktelement 12 an der Schulter 10 angeordnet ist. Das Kontaktelement 12 verfügt dabei über eine Kontaktfläche 16, die im gezeigten Ausführungsbeispiel eben ausgebildet ist und an der beispielsweise ein Kraftübertragungselement anliegt, durch dass die von dem nicht dargestellten wenigstens einen Federelement aufgebrachte Kraft in das Kontaktelement 12 eingeleitet wird.

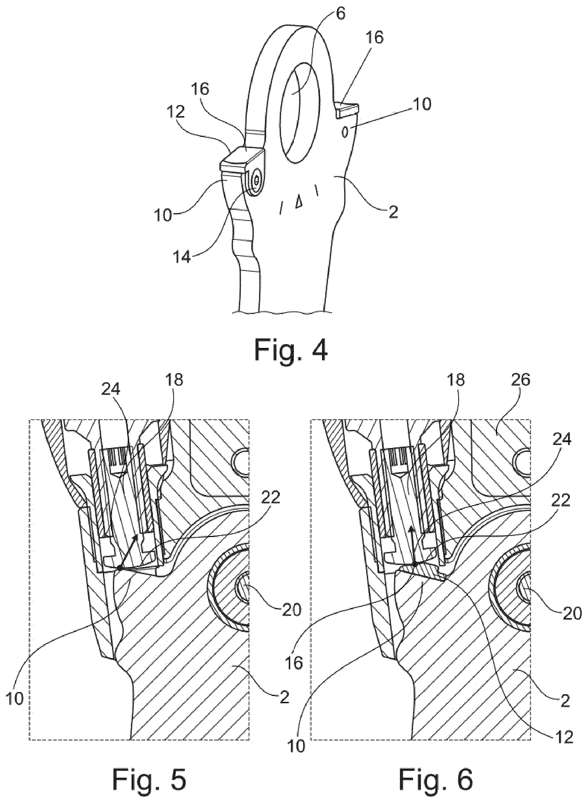

Figur 4 zeigt den oberen Teil des Fußbügels 2 aus den Figuren 1, 2 und 3 in einer schematischen, dreidimensionalen Ansicht. Man erkennt die Öffnung 6, die beiden Schultern 10 sowie das über die Schraube 14 angeordnete Kontaktelement 12. Anders als in Figur 3 ist jedoch hier die Kontaktfläche 16 konkav ausgebildet und bildet in diesem Fall eine Längsrille, die sich in radialer Richtung bezüglich einer durch die Öffnung 6 hindurchzuführenden Schwenkachse erstreckt. Auf diese Weise ist ein möglichst guter Kontakt zwischen dem nicht dargestellten Kraftübertragungselement und der Kontaktfläche des Kontaktelementes gewährleistet.

Diese Situation ist in den Figuren 5 und 6 dargestellt. Man erkennt ein Kraftübertragungselement 18, durch das eine von einem nicht dargestellten Federelement ausgeübte Kraft auf eine Schulter 10 eines Fußbügels 2 aus dem Stand der Technik aufgebracht wird. Wird der Fußbügel 2 um die Schwenkachse 20 herum im Uhrzeigersinn verschwenkt, wird sich die Schulter 10 nach oben bewegen, das Kraftübertragungselement 18 ebenfalls nach oben verschieben und dadurch das nicht dargestellte Federelement komprimieren. Bei der Darstellung in Figur 5 handelt es sich nicht um ein erfindungsgemäßes Gelenk, da der Fußbügel 2 nicht mit einem Kontaktelement 12 ausgebildet ist. Vielmehr bildet die Schulter 10 die Kontaktfläche, die mit einem konvex ausgebildeten Kontaktbereich 22 des Kraftübertragungselementes 18 in mechanischen Kontakt kommt. Durch die Formgebung der Kontaktfläche der Schulter 10 sowie des Kontaktbereiches 22 des Kraftübertragungselementes 18 wird beim Verschwenken des Fußbügels 2 im Uhrzeigersinn um die Schwenkachse 20 herum eine Kraft entlang des Pfeiles 24 auf das Kraftübertragungselement 18 aufgebracht. Diese Kraft und der Pfeil 24 sind jedoch nicht parallel zu einer Längserstreckung Kraftübertragungselementes 18, die der Verschieberichtung des Kraftübertragungselementes 18 und der Kompressionsrichtung des nicht gezeigten Federelementes entspricht.

Figur 6 zeigt die gleiche Situation mit einem Fußbügel 2 für ein Gelenk gemäß einem Ausführungsbeispiel der vorliegenden Erfindung. An der Schulter 10 ist das Kontaktelement 12 angeordnet, das eine dem Kontaktbereich 22 des Kraftübertragungselementes 18 entsprechend geformte Kontaktfläche 16 aufweist. Unabhängig von der Position und Orientierung des Fußbügels 2 relativ zum Unterschenkelelement 26 wird beim Verschwenken des Fußbügels 2 um die Schwenkachse 20 im Uhrzeigersinn eine Kraft auf das Kraftübertragungselement 18 aufgebracht, die entlang des Pfeiles 24 verläuft und somit immer parallel zur Verschieberichtung des Kraftübertragungselementes 18 und damit zur Kompressionsrichtung des nicht gezeigten Federelementes aufgebracht wird.

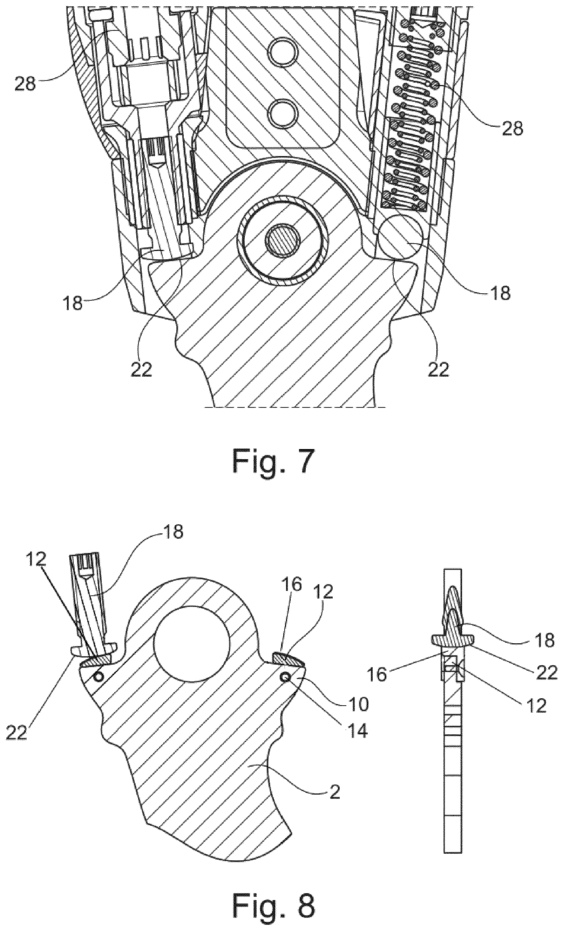

Figur 7 zeigt schematisch unterschiedliche Formen von Federelementen 28 und unterschiedliche Formen von Kraftübertragungselementen 18. Während im linken Bereich das Kraftübertragungselement 18 aus den Figuren 5 und 6 dargestellt ist, das über den bereits beschriebenen Kontaktbereich 22 verfügt, ist im rechten Beispiel das Kraftübertragungselement 18 in Form einer Kugel ausgebildet, wobei in diesem Fall der Kontaktbereich 22 eine deutlich kleinere Fläche aufweist. Auch wenn im in Figur 7 gezeigten Ausführungsbeispiel keine Kontaktelemente 12 dargestellt sind, sind sie doch vorhanden und nur aus Übersichtlichkeitsgründen nicht zeichnerisch dargestellt.

Figur 8 zeigt in der linken Darstellung den Fußbügel 2, an dem zwei Kontaktelemente 12 angeordnet sind, die mittels der Schraube 14 an der Schulter 10 positioniert werden. Man erkennt, dass die Kontaktelemente 12 eine gewölbt ausgebildete Kontaktfläche 16 aufweisen, die mit der Wölbung des Kontaktbereiches 22 des Kraftübertragungselementes 18 korrespondiert. Im linken Teil der Figur 8 entspricht dies der Darstellung aus Figur 6. Im rechten Teil der Figur 8 ist eine Darstellung gezeigt, die der Darstellung aus den Figuren 2 und 3 entspricht. Man erkennt auch hier das Kraftübertragungselement 18, das einen gewölbten Kontaktbereich 22 aufweist. Der im rechten Teil der Figur 8 gezeigte Schnitt entspricht dem einer Frontalebene und man erkennt, dass auch die Kontaktfläche 16 des Kontaktelementes 12 in dieser Richtung gewölbt ausgebildet ist. Der Kontakt zwischen dem Kontaktbereich 22 des Kraftübertragungselementes 18 und der Kontaktfläche 16 des Kontaktelementes 12 ist durch diese Ausgestaltung immer linienförmig, so dass die auftretenden Drücke an den jeweiligen Stellen möglichst gering gehalten werden.

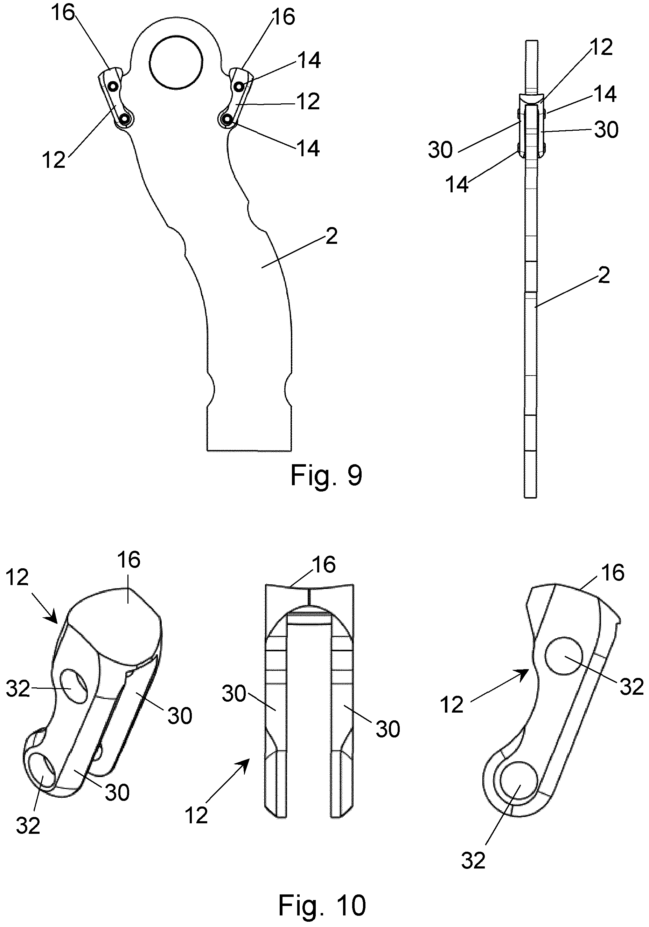

Figur 9 zeigt eine weitere Ausgestaltung eines Fußbügels 2, an dem sich zwei Kontaktelemente 12 befinden. Diese verfügen über jeweils eine Kontaktfläche 16 und sind im gezeigten Ausführungsbeispiel durch jeweils zwei Schrauben 14 am Fußbügel 2 befestigt. Alternativ dazu können die Kontaktelemente 12 auch durch zwei Stifte oder Bolzen oder durch eine Schraube 14 und einen Stift oder Bolzen am Fußbügel 2 befestigt sein. In der rechten Darstellung von Figur 9 ist der Fußbügel 2 mit einem der Kontaktelemente 12 dargestellt. Das Kontaktelement 12 verfügt über zwei Schenkel 30, die auf beiden Seiten des Fußbügels 2 angeordnet sind. Die beiden Schenkel 30 sowie der Fußbügel 2 verfügen jeweils über Bohrungen oder Ausnehmungen, die in der gezeigten Ausführungsform in Überdeckung zueinander gebracht wurden, sodass die Schrauben 14 hindurchgesteckt oder hindurchgeschraubt werden können. Auf diese Weise entsteht eine symmetrische Befestigung des Kontaktelementes 12 am Fußbügel 2, wodurch eine besonders gleichmäßige Belastung und damit eine hohe Verschleißfestigkeit erreicht wird.

Figur 10 zeigt ein Kontaktelement 12 in drei unterschiedlichen Darstellungen. Es verfügt über die Kontaktfläche 16, die leicht konkav ausgebildet ist, wie insbesondere in der mittleren Darstellung von Figur 10 zu erkennen ist. Das Kontaktelement 12 verfügt über die beiden Schenkel 30, die jeweils über zwei Bohrungen 32 verfügen.

### Bezugszeichenliste

- 2 -: Fußbügel
- 4 -: Befestigungselement
- 6 -: Öffnung
- 8 -: Markierung
- 10 -: Schulter

- 12 -: Kontaktelement
- 14 -: Schraube
- 16 -: Kontaktfläche
- 18 -: Kraftübertragungselement
- 20 -: Schwenkachse

- 22 -: Kontaktbereich
- 24 -: Pfeil
- 26 -: Unterschenkelelement
- 28 -: Federelement
- 30 -: Schenkel
- 32 -: Bohrung

## Patentansprüche

1. Gelenk für eine orthopädietechnische Einrichtung, wobei das Gelenk
- ein erstes Element mit wenigstens einem Anschlagselement und
- ein zweites Element aufweist, das an dem ersten Element verschwenkbar gelagert ist und wenigstens eine Kontaktfläche (16) aufweist, die mit dem wenigstens einen Anschlagselement in Kontakt bringbar ist, indem das zweite Element relativ zu dem ersten Element verschwenkt wird, wobei das wenigstens eine Anschlagselement dann einem weiteren Verschwenken des zweiten Elementes relativ zum ersten Element eine Kraft entgegensetzt,
wobei die orthopädietechnische Einrichtung eine Knöchelorthese ist, wobei das erste Element ein Unterschenkelelement (26) und das zweite Element ein Fußbügel (2) ist, wobei das Anschlagselement federbelastet oder gedämpft ist und die Kraft überwindbar ausgestaltet ist, so dass ein weiteres Verschwenken möglich ist,
**dadurch gekennzeichnet, dass** an dem zweiten Element wenigstens ein Kontaktelement (12) lösbar befestigt ist, das die wenigstens eine Kontaktfläche (16) aufweist.

2. Gelenk nach einem der vorstehenden Ansprüche, **dadurch gekennzeichnet, dass** an dem zweiten Element wenigstens zwei Kontaktelemente (12) lösbar befestigt sind und das Gelenk wenigstens zwei Anschlagselemente aufweist, wobei die Kontaktelemente (12) jeweils wenigstens eine Kontaktfläche (16) aufweisen, die mit jeweils wenigstens einem Anschlagselement in Kontakt bringbar sind.

3. Gelenk nach einem der vorstehenden Ansprüche, **dadurch gekennzeichnet, dass** jedes Anschlagselement wenigstens ein Kraftübertragungselement (18) mit einem Kontaktbereich (22) aufweist, der an der Kontaktfläche (16) des jeweiligen Kontaktelementes (12) anliegt, wenn die Kontaktfläche (16) des Kontaktelementes (12) mit dem Anschlagselement in Kontakt kommt.

4. Gelenk nach einem der vorstehenden Ansprüche, **dadurch gekennzeichnet, dass** die wenigstens einen Kontaktfläche (16) derart geformt ist, dass die von dem wenigstens einen Anschlagselement dem weiteren Verschwenken entgegengesetzte Kraft unabhängig von einer Position des ersten Elementes relativ zu dem zweiten Element immer senkrecht auf der Kontaktfläche (16) steht.

5. Gelenk nach einem der vorstehenden Ansprüche, **dadurch gekennzeichnet, dass** das wenigstens eine Kontaktelement (12) aus einem härteren Material als das zweite Element, vorzugsweise aus gehärtetem Stahl, hergestellt ist.

6. Gelenk nach einem der Ansprüche 1 bis 4, **dadurch gekennzeichnet, dass** das wenigstens eine Kontaktelement (12) aus einem weicheren Material als das zweite Element, vorzugsweise aus Eladur, hergestellt ist.

7. Gelenk nach Anspruch 6, **dadurch gekennzeichnet, dass** das wenigstens eine Kontaktelement (12) ein elastisches Material enthält.

8. Gelenk nach einem der vorstehenden Ansprüche, **dadurch gekennzeichnet, dass** die Kontaktfläche (16) konkav ausgebildet ist.

9. Gelenk nach einem der Ansprüche 3 bis 7, **dadurch gekennzeichnet, dass** der Kontaktbereich (22) konvex ausgebildet ist und vorzugsweise einen Krümmungsradius aufweist, der einem Krümmungsradius der Kontaktfläche (16) des Kontaktelementes (12) entspricht.

10. Gelenk nach einem der vorstehenden Ansprüche, **dadurch gekennzeichnet, dass** das wenigstens eine Kontaktelement (12) an dem zweiten Element angeschraubt ist.

11. Gelenk nach einem der vorstehenden Ansprüche, **dadurch gekennzeichnet, dass** das wenigstens eine Kontaktelement (12) und das zweite Element derart korrespondierend ausgebildet und geformt sind, dass das Kontaktelement (12) nur in einer Orientierung an dem zweiten Element befestigbar ist.

12. Gelenk nach einem der vorstehenden Ansprüche, **dadurch gekennzeichnet, dass** das Gelenk derart ausgebildet ist, dass das Kontaktelement (12) in zumindest einer Orientierung und/oder Position des ersten Elementes relativ zu dem zweiten Element sichtbar ist, ohne das Gelenk zu demontieren.

## Claims

1. A joint for an orthopedic device, wherein the joint comprises
- a first element with at least one end stop element and
- a second element which is mounted on the first element such that it can be swivelled and has at least one contact surface (16) that can be brought into contact with the at least one end stop element by swivelling the second element relative to the first element, wherein the at least one end stop element then counters a further swivelling of the second element relative to the first element with a force,
wherein the orthopedic device is an ankle orthosis, wherein the first element is a lower leg element (26) and the second element is a foot stirrup (2), wherein the end stop element is spring-loaded or damped and the force can be overcome, so that further swivelling is possible,
**characterized in that** at least one contact element is fixed on the second element such that it can be detached, said contact element having at least one contact surface.

2. The joint according to one of the preceding claims, **characterized in that** at least two contact elements (12) are fixed on the second element such that they can be detached and the joint comprises at least two end stop elements, wherein the contact elements (12) each have at least one contact surface (16) that can be brought into contact with at least one end stop element.

3. The joint according to one of the preceding claims, **characterized in that** each end stop element comprises at least one force transmission element (18) with a contact area (22) that rests on the contact surface (16) of the respective contact element (12) when the contact surface (16) of the contact element (12) comes into contact with the end stop element.

4. The joint according to one of the preceding claims, **characterized in that** the at least one contact surface (16) is formed in such a way that the force that counters further swivelling applied by the at least one stop element is always perpendicular to the contact surface (16) irrespective of a position of the first element relative to the second element.

5. The joint according to one of the preceding claims, **characterized in that** the at least one contact element (12) is made of a harder material than the second element, preferably of hardened steel.

6. The joint according to one of the claims 1 to 6, **characterized in that** the at least one contact element (12) is made of a softer material than the second element, preferably Eladur.

7. The joint according to claim 8, **characterized in that** the at least one contact element (12) contains an elastic material.

8. The joint according to one of the preceding claims, **characterized in that** the contact surface (16) is designed to be concave.

9. The joint according to one of the claims 5 to 9, **characterized in that** the contact area (22) is designed to be convex and preferably has a radius of curvature that corresponds to a radius of curvature of the contact surface (16) of the contact element (12).

10. The joint according to one of the preceding claims, **characterized in that** the at least one contact element (12) is screwed onto the second element.

11. The joint according to one of the preceding claims, **characterized in that** the at least one contact element (12) and the second element are correspondingly designed and formed in such a way that the contact element (12) can only be fixed to the second element in a single orientation.

12. The joint according to one of the preceding claims, **characterized in that** the joint is designed in such a way that the contact element (12) is visible in at least one orientation and/or position of the first element relative to the second element without dismantling the joint.

## Revendications

1. Articulation pour un dispositif orthopédique, ladite articulation comportant
- un premier élément muni d'au moins un élément de butée, et
- un deuxième élément monté de manière pivotante sur le premier élément et présentant au moins une surface de contact (16) apte à être mise en contact avec ledit au moins un élément de butée par pivotement du deuxième élément par rapport au premier élément, ledit au moins un élément de butée opposant alors une force à tout pivotement supplémentaire du deuxième élément par rapport au premier élément,
le dispositif orthopédique étant une orthèse de cheville, le premier élément étant un élément de jambe (26) et le deuxième élément étant un étrier de pied (2), l'élément de butée étant sollicité par ressort ou étant amorti et la force étant conçue de manière à pouvoir être surmontée, de sorte qu'un pivotement supplémentaire est possible,
**caractérisée en ce qu'**au moins un élément de contact (12), présentant ladite au moins une surface de contact (16), est fixé de manière amovible au deuxième élément.

2. Articulation selon la revendication précédente,
**caractérisée en ce qu'**au moins deux éléments de contact (12) sont fixés de manière amovible au deuxième élément, et **en ce que** l'articulation comporte au moins deux éléments de butée, les éléments de contact (12) présentant chacun au moins une surface de contact (16) apte à être mise en contact avec au moins un élément de butée respectif.

3. Articulation selon l'une des revendications précédentes,
**caractérisée en ce que** chaque élément de butée comporte au moins un élément de transmission de force (18) muni d'une zone de contact (22) qui vient en appui contre la surface de contact (16) de l'élément de contact (12) respectif lorsque la surface de contact (16) de l'élément de contact (12) entre en contact avec l'élément de butée.

4. Articulation selon l'une des revendications précédentes,
**caractérisée en ce que** ladite au moins une surface de contact (16) est formée de telle sorte que la force exercée par ledit au moins un élément de butée pour s'opposer au pivotement supplémentaire soit toujours perpendiculaire à la surface de contact (16), quelle que soit la position du premier élément par rapport au deuxième élément.

5. Articulation selon l'une des revendications précédentes,
**caractérisée en ce que** ledit au moins un élément de contact (12) est réalisé en un matériau plus dur que celui du deuxième élément, de préférence en acier trempé.

6. Articulation selon l'une des revendications 1 à 4,
**caractérisée en ce que** ledit au moins un élément de contact (12) est réalisé en un matériau plus tendre que celui du deuxième élément, de préférence en Eladur.

7. Articulation selon la revendication 6,
**caractérisée en ce que** ledit au moins un élément de contact (12) contient un matériau élastique.

8. Articulation selon l'une des revendications précédentes,
**caractérisée en ce que** la surface de contact (16) est de forme concave.

9. Articulation selon l'une des revendications 3 à 7,
**caractérisée en ce que** la zone de contact (22) est de forme convexe et présente de préférence un rayon de courbure qui correspond au rayon de courbure de la surface de contact (16) de l'élément de contact (12).

10. Articulation selon l'une des revendications précédentes,
**caractérisée en ce que** ledit au moins un élément de contact (12) est vissé sur le deuxième élément.

11. Articulation selon l'une des revendications précédentes,
**caractérisée en ce que** ledit au moins un élément de contact (12) et le deuxième élément sont conçus et formés de manière correspondante, de sorte que l'élément de contact (12) ne puisse être fixé au deuxième élément que selon une seule orientation.

12. Articulation selon l'une des revendications précédentes,
**caractérisée en ce que** l'articulation est conçue de telle sorte que l'élément de contact (12) soit visible selon au moins une orientation et/ou une position du premier élément par rapport au deuxième élément, sans qu'il soit nécessaire de démonter l'articulation.
